# FASCICULE DE BREVET EUROPEEN

(11) **EP 3 283 064 B1**
(45) Date de publication et mention de la délivrance du brevet: **08.05.2019**
(21) Numéro de dépôt: 16729310.9
(22) Date de dépôt: 14.04.2016
(51) Int. Cl.: A61K 31/165, A61K 31/197, A61K 31/216, A61K 31/4402, A61K 31/4406, A61K 31/495, A61K 31/50, A61K 31/15, A61P 21/00, A61P 21/06

(54) **DÉRIVES UTILES DANS LE TRAITEMENT DE L'ATROPHIE MUSCULAIRE**
IN DER BEHANDLUNG VON MUSKELATROPHIE VERWENDETE DERIVATE
DERIVATIVES USED IN THE TREATMENT OF MUSCLE ATROPHY

(30) Priorité: 16.04.2015 FR 1553410
(43) Date de publication de la demande: 21.02.2018
(73) Titulaire: Metabrain Research, 91380 Chilly Mazarin (FR)
(72) Inventeur: RAYNAL, Sophie N., 75019 Paris (FR); KERGOAT, Micheline R., 91940 Les Ulis (FR); AUTIER, Valérie, 91190 Gif Sur Yvette (FR); CHARON, Christine G., 91940 Gometz Le Chatel (FR); DURAND, Jean-Denis, 93100 Montreuil Sous Bois (FR); LEPIFRE, Franck F., 91400 Saclay (FR); AUDET, Annick M., 91630 Leudeville (FR)
(74) Mandataire: Salmon, Victoria Beatrice
(86) Numéro de dépôt international: PCT/FR2016/050864
(87) Numéro de publication internationale: WO 2016/166480

(56) Documents cités:
- WO-A1-98/09940
- WO-A1-2010/011302
- FR-A1- 2 204 406
- US-A1- 2002 161 050

### SEQUENCE LISTING

<110> METABRAIN RESEARCH
<120> Dérivés utiles dans le traitement de l'atrophie musculaire
<130> 1H308550 0004 WO
<160> 6
<170> PatentIn version 3.5
<210> 1
   <211> 22
   <212> DNA
   <213> artificial sequence
<220>
   <223> amorce
<400> 1
   gagtctgact ttctaatgca ag 22
<210> 2
   <211> 20
   <212> DNA
   <213> artificial sequence
<220>
   <223> amorce
<400> 2
   tgttgtagga gtcttgacgg 20
<210> 3
   <211> 20
   <212> DNA
   <213> artificial sequence
<220>
   <223> amorce
<400> 3
   agagtcggca agtctgtgct 20
<210> 4
   <211> 19
   <212> DNA
   <213> artificial sequence
<220>
   <223> amorce
<400> 4
   gtgaggcctt tgaaggcag 19
<210> 5
   <211> 20
   <212> DNA
   <213> artificial sequence
<220>
   <223> amorce
<400> 5
   ctctagactt cgagcaggag 20
<210> 6
   <211> 19
   <212> DNA
   <213> artificial sequence
<220>
   <223> amorce
<400> 6
   ggtaccacca gacagcact 19

## Description

La présente invention concerne des dérivés utiles pour le traitement des maladies liées à l'atrophie musculaire.

### Champ de l'invention

On note aujourd'hui un manque évident de nouveaux traitements permettant de retarder l'apparition et la progression de troubles et maladies liées au vieillissement qui s'accompagnent d'une diminution de la force ou puissance musculaire comme la sarcopénie alors que les problèmes de mobilité liés à l'âge sont des problèmes très graves avec des conséquences de grande envergure en matière de santé.

Les personnes âgées atteintes de certains troubles ou maladies métaboliques sont confrontées la plupart du temps à une perte de masse maigre due au moins en partie, à une réduction de la synthèse des protéines musculaires (comme la sarcopénie), une diminution d'apport nutritionnel, ou la présence d'une inflammation. Une diminution de la masse maigre du corps est connue pour être associée à la fatigue, une diminution de la capacité à exécuter les tâches nécessaires à la vie quotidienne, un risque accru de fractures osseuses, une augmentation des chutes et des hospitalisations, une altération du métabolisme (tolérance au glucose, sensibilité à l'insuline) et de la capacité cognitive ainsi qu'une diminution de la qualité de vie (Frontera WR et al., 1991; Baumgartner et al., 1998; Lloyd BD et al., 2009). Contrairement à la cachexie, les patients souffrant de sarcopénie peuvent avoir un poids stable, mais montrer une nette perte de masse musculaire, tandis que typiquement dans le même temps la masse de graisse augmente.

De nombreux facteurs influencent la diminution de la masse musculaire. On constate essentiellement, une résistance anabolique du muscle squelettique à la synthèse protéique comme on peut le voir lors d'une immobilisation qui peut être améliorée au moins en partie par des exercices de résistance et l'apport de suppléments alimentaires (Farnfield MM et al. 2012). Il a également été démontré qu'une perte d'innervation et des dommages oxydatifs pouvaient jouer un rôle important (Chai RJ et al., 2011; O'Neill ED et al., 2010) et conduire à une atrophie musculaire impliquant différentes voies de signalisation cellulaire elle-même conduisant à la mort cellulaire programmée (apoptose), l'augmentation de la dégradation des protéines, ou même une diminution de l'activation des cellules responsables de la régénération du muscle. La sarcopénie est donc le résultat d'un déséquilibre entre la dégradation des protéines et leur synthèse, où la contribution exacte de chacun des facteurs cités ci-dessus varie selon le modèle ou cas étudié. Certains systèmes protéolytiques ont été décrits comme participant à la dégradation musculaire comme les protéases activées par le calcium telles que la calpaïne et les caspases; le système ubiquitine-protéasome (Hasselgren PO et al., 2002; Purintrapiban J et al., 2003). Parmi les différentes cibles identifiées lors du développement de la sarcopénie, l'atrogin-1 (également connue sous le nom MAFbx) et MURF-1 ont été trouvées augmentées et Akt / mTOR / S6K diminuées.

La myostatine, produite de façon autocrine par les muscles eux-mêmes, représente également un facteur particulièrement important, car cette protéine agit à la fois en stimulant la protéolyse et en inhibant la protéosynthèse. Alors que le vieillissement s'accompagne de modifications hormonales importantes, on constate notamment une augmentation de sécrétion de myostatine (Léger et al., 2008) dont l'expression dans des muscles adultes augmente avec le degré d'atrophie. La myostatine, également connue comme facteur de croissance et de différenciation-8 (GDF-8) possède toutes les caractéristiques structurales communes aux protéines de la famille TGF-β: un amino-terminal hydrophobe qui agit comme un signal de sécrétion, neuf résidus de cystéine invariants, et un site de traitement protéolytique de type furine "RXXR". Le clivage protéolytique de la protéine donne lieu à un domaine C-terminal qui forme un homodimère qui est la forme biologiquement active de la myostatine (Thies et al., 2001). Les alignements de séquences aminoacides de la myostatine provenant d'espèces de vertébrés multiples montrent que la protéine est hautement conservée (100% d'identité) entre l'homme, le singe, la vache, le chien, la souris, le rat, la dinde et le poulet (McPherron et al., 1997). Son expression est limitée principalement au muscle squelettique et le tissu adipeux, où elle joue le rôle de régulateur négatif du développement du muscle squelettique (Lee LS, 2010, 10: 183-194).

Il a été montré que des souris et des bovins génétiquement déficients en myostatine présentent une augmentation spectaculaire de la masse musculaire squelettique, soutenant ainsi le rôle de la myostatine dans la suppression de la croissance musculaire (Wolfman et al., 2003). Chez certaines races bovines, l'hypertrophie musculaire est due à une mutation faux-sens dans le troisième exon du gène de la myostatine bovine (Bass et al., 1999) qui s'accompagne d'une augmentation à la fois du nombre de cellules ou croissance hyperplasique, et de la taille des cellules, ou croissance hypertrophique, se traduisant ainsi par de plus grandes et plus lourdes myofibres.

L'augmentation de la masse musculaire squelettique et la force sont également associées à des adaptations métaboliques qui influencent positivement la composition du corps, la dépense énergétique, l'homéostasie du glucose et les besoins en insuline. Des données pharmacologiques et génétiques indiquent que la myostatine régule le métabolisme énergétique et que son inhibition peut atténuer de manière significative la progression de maladies métaboliques comme l'obésité et le diabète. Par exemple, les souris génétiquement déficientes en myostatine présentent une faible accumulation de graisse corporelle (McPherron et al., 2002) par rapport aux souris de type sauvage du même âge, liée à une réduction du nombre d'adipocytes et de leur taille, démontrant ainsi le rôle important de la myostatine dans l'adipogenèse, et la myogenèse.

Les traitements expérimentaux actuels pour traiter la Sarcopénie reposent sur des approches nutritionnelles, la pratique de l'exercice physique, l'utilisation de stimulants de l'appétit ou de composés anabolisants comme la testostérone, mais les effets de ces traitements ne sont pas satisfaisants (Morley et al., 2007) et peuvent entrainer des effets secondaires.

La présente invention concerne des dérivés et des compositions pharmaceutiques les comprenant pour leur utilisation pour le traitement et /ou la prévention de l'atrophie musculaire chez le mammifère.

### Description détaillée de l'invention

La présente invention concerne donc des dérivés de formule générale I suivante : 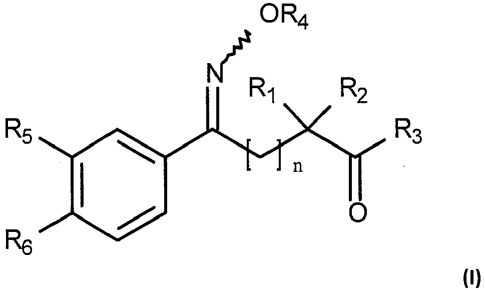 dans laquelle
- **R¹** et **R²** représentent indépendamment l'un de l'autre un atome d'hydrogène ou un groupe alkyle en C₁-C₆, avantageusement méthyle ;
- n est un nombre entier compris entre 1 et 2, avantageusement n=1 ;
- **R³** représente
   - un groupe -OH;
   - un groupe-O(alkyle en C₁-C₆), avantageusement-OEt ou -OiPr;
   - un groupe-O(cycloalkyle en C₃-C₆), avantageusement-O-cyclopentyle;
   - un groupe-O(hétérocyclique en C₃-C₇), avantageusement-O-tétrahydropyran-4-yl;
   - un groupe -O-hétéroaryle, avantageusement -O-pyridyle ;
   - un groupe -O-aryle, avantageusement -O-phényle ;
   - un groupe - O(alkyle en C₁-C₆)aryle, avantageusement -O-benzyle ;
   - un groupe -N**R⁷R⁸**, dans lequel
      **R⁷** représente un atome d'hydrogène ou un groupe alkyle en C₁-C₆ **et R⁸** représente
      - un atome d'hydrogène;
      - un groupe hétéroaryle, avantageusement contenant un ou plusieurs atomes d'azote, en particulier un groupe pyridazinyle ou un groupe pyridyle;
      - un groupe alkyle en C₁-C₆, avantageusement un groupe éthyle, éventuellement substitué par un groupe -N**R⁹R¹⁰** dans lequel **R⁹** et **R¹⁰** représentent indépendamment l'un de l'autre un atome d'hydrogène ou un groupe alkyle en C₁-C₆, avantageusement un groupe méthyle ;
      - un groupe alkyle en C₁-C₆, avantageusement un groupe éthyle, éventuellement substitué par un groupe -O**R¹¹** dans lequel **R¹¹** représente un atome d'hydrogène ou un groupe alkyle en C₁-C₆, avantageusement un groupe méthyle ;
      **ou R⁷** et **R⁸** forment avec l'atome d'azote qui les portent un hétérocycle, en particulier une pyrrolidine, une pipéridine, une morpholine, une pipérazine, l'hétérocycle étant éventuellement substituée par un groupe alkyle en C₁-C₆, avantageusement un groupe méthyle, en particulier **R⁷** et **R⁸** forment une méthylpipérazinyle ;
- **R⁴** représente un atome d'hydrogène ou un groupe alkyle en C₁-C₆, en particulier -Me ou -Et, plus particulièrement -Et ;
- **R⁵** et **R⁶** représentent indépendamment l'un de l'autre un hydrogène, un atome d'halogène, avantageusement Cl, ou un groupe choisi parmi :
   un groupe -CN ;
   un groupe -OH ;
   un groupe -O(alkyle en C₁-C₆), avantageusement -OMe, le groupe alkyle étant éventuellement substitué par un ou plusieurs atomes d'halogène, avantageusement F, tel que par exemple -OCF₃ ou -OCHF₂;
   un groupe alkyle en C₁-C₆, avantageusement méthyle, éventuellement substitué par un ou plusieurs atomes d'halogène, avantageusement F, tel que par exemple -CF₃, un groupe -O(cycloalkyle en C₃-C₆), avantageusement O-cyclopropyle ;
ou un énantiomère, diastéréoisomère, hydrate, solvate, tautomère, mélange racémique ou sel pharmaceutiquement acceptable de celui-ci
pour utilisation à titre de médicament destiné au traitement et/ou à la prévention de l'atrophie musculaire chez les mammifères et/ou pour limiter l'atrophie musculaire chez les mammifères et/ou pour favoriser l'accroissement musculaire des mammifères, faisant de l'exercice et visant à augmenter la masse et la qualité musculaire, en prévention de l'apparition de symptômes sarcopéniques liés à l'âge ou en rééducation suite à une perte musculaire,
l'atrophie musculaire étant liée à l'âge et/ou aux conséquences d'un traitement médicamenteux et/ou à une immobilisation et/ou à la cachexie.

En particulier l'atrophie musculaire est liée à l'âge, comme la pré-sarcopénie, la sarcopénie ou la sarcopénie sévère. L'atrophie musculaire peut aussi être liée aux conséquences d'un traitement médicamenteux, comme par exemple un traitement anti-cancéreux. L'atrophie musculaire peut également être liée à une immobilisation, en particulier quelle qu'en soit la cause, par exemple due à la faiblesse liée à l'âge, à un accident ou à une opération chirurgicale, comme par exemple la pose d'une prothèse du genou ou de la hanche. L'atrophie musculaire peut enfin être liée à la cachexie, en particulier quelle qu'en soit la cause, par exemple due à l'anorexie mentale, au cancer, à l'insuffisance cardiaque, à l'insuffisance hépatique, à l'insuffisance rénale, à la tuberculose ou au SIDA.

En particulier le mammifère peut être un animal ou l'homme, de façon avantageuse il s'agit de l'homme.

Selon la présente invention, les dérivés de formule (I) présentent ainsi une activité pour le traitement et /ou la prévention de l'atrophie musculaire, en particulier de l'atrophie musculaire liée à l'âge comme la pré-sarcopénie, la sarcopénie, la sarcopénie sévère, et/ou liée aux conséquences d'un traitement médicamenteux comme par exemple un traitement anti-cancéreux, et/ou liée à une immobilisation, en particulier quelle qu'en soit la cause, comme par exemple la faiblesse liée à l'âge, un accident, une opération chirurgicale comme par exemple la pose d'une prothèse du genou ou de la hanche, la cachexie, et de façon générale les pathologies liées à l'atrophie musculaire chez le mammifère.

Les dérivés de formule (I) sont également utiles pour favoriser l'accroissement musculaire des mammifères, avantageusement de l'homme, faisant de l'exercice et visant à augmenter la masse et la qualité musculaire, en prévention par exemple de l'apparition de symptômes sarcopéniques liés à l'âge.

Les inventeurs ont découvert que les dérivés selon la présente invention permettaient d'inhiber l'expression génique de la myostatine, d'augmenter la synthèse protéique dans les cellules musculaires et/ou d'augmenter le diamètre des myotubes de cellules C2C12.

La présente invention concerne de plus l'utilisation d'un dérivé de formule (I) selon l'invention tels que définis ci-dessus pour la fabrication d'un médicament destiné au traitement et/ou à la prévention de l'atrophie musculaire chez les mammifères et/ou pour limiter l'atrophie musculaire chez les mammifères et/ou pour favoriser l'accroissement musculaire des mammifères faisant de l'exercice et visant à augmenter la masse et la qualité musculaire, en prévention de l'apparition de symptômes sarcopéniques liés à l'âge ou en rééducation suite à une perte musculaire.

La quantité efficace sera adaptée selon la nature et la sévérité de la pathologie à traiter, la voie d'administration et aussi le poids et l'âge du sujet. En générale l'unité de doses variera entre 0,5 mg et 2000 mg par jour, en une prise ou plusieurs, avantageusement entre 1 et 1000 mg lorsque le sujet est un homme.

Dans un mode de réalisation avantageux, les dérivés de formule (I) utiles dans le cadre de la présente invention sont tels que **R⁴** représente un groupe -(alkyle en C₁-C₆), avantageusement -Et.

Dans un autre mode de réalisation avantageux, les dérivés de formule (I) utiles dans le cadre de la présente invention sont tels que **n** = 1;

Dans encore un autre mode de réalisation avantageux, les dérivés de formule (I) utiles dans le cadre de la présente invention sont tels que les groupes **R⁵** et / ou **R⁶** représentent un atome d'halogène, avantageusement un atome de chlore.

Dans encore un autre mode de réalisation avantageux, les dérivés de formule (I) utiles dans le cadre de la présente invention sont tels que **R⁵** et **R⁶** représentent indépendamment l'un de l'autre un atome d'halogène, avantageusement Cl ; particulièrement **R⁵** et **R⁶** sont des chlores, et **R⁴** représente un groupe alkyle en C₁-C₆, en particulier Et.

Dans un autre mode de réalisation avantageux, les dérivés de formule (I) utiles dans le cadre de la présente invention sont tels que **R¹** et **R²** représentent tous les deux un atome d'hydrogène.

Dans encore un autre mode de réalisation avantageux, les dérivés de formule (I) utiles dans le cadre de la présente invention sont tels que **R³** représente un groupe -OH ou un groupe - O(alkyle en C₁-C₆), avantageusement-OEt ou -OiPr.

En particulier les dérivés de formule (I) utiles dans le cadre de la présente invention sont choisis parmi les composés suivants.
**(1)** acide 4-(3,4-dichlorophényle)-4-éthoxyimino-butanoique;
**(2)** acide 4-(3,4-dichlorophényle)-4-méthoxyimino-butanoique;
**(3)** 4-(3,4-dichlorophényle)-4-éthoxyimino-butanoate d'isopropyle;
**(4)** acide 5-(3,4-dichlorophényle)-5-éthoxyimino-pentanoique;
**(5)** 4-(3,4-dichlorophényle)-4-éthoxyimino-*N*-(2-pyridyle)butanamide;
**(6)** 4-(3,4-dichlorophényle)-4-éthoxyimino-*N*-(3-pyridyle)butanamide;
**(7)** 4-(3,4-dichlorophényle)-4-éthoxyimino-*N*-pyridazine-3-yl-butanamide;
**(8)** 4-(3,4-dichlorophényle)-*N*-(2-diméthylaminoéthyle)-4-éthoxyimino-butanamide;
**(9)** (4E)-4-(3,4-dichlorophényle)-4-éthoxyimino-N-(3-hydroxypropyle)butanamide ;
**(10)** 4-(3,4-dichlorophényle)-4-éthoxyimino-1-(4-méthylpiperazine-1-yl)butan-1-one;

En particulier les composés suivants **1** à **10** qui sont couverts par la formule générale (I) sont décrits dans l'art antérieur pour d'autres applications:

| **N°** | **Structure chimique** | **Nom chimique** |
|---|---|---|
| **1** | 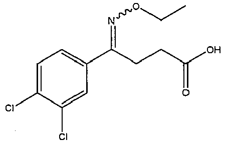 | acide 4-(3,4-dichlorophényle)-4-éthoxyimino-butanoique |
| **2** | 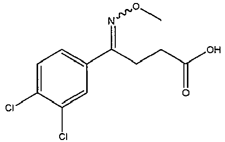 | acide 4-(3,4-dichlorophényle)-4-méthoxyimino-butanoique |
| **3** | 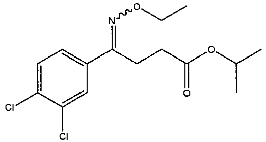 | 4-(3,4-dichlorophényle)-4-éthoxyimino-butanoate d'isopropyle |
| **4** | 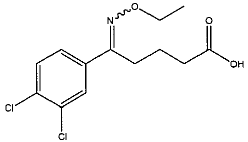 | acide 5-(3,4-dichlorophényle)-5-éthoxyimino-pentanoique |
| **5** | 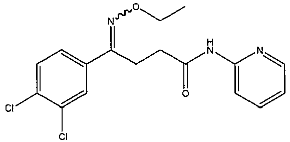 | 4-(3,4-dichlorophényle)-4-éthoxyimino-*N*-(2-pyridyle)butanamide |

| | | |
|---|---|---|
| **6** | 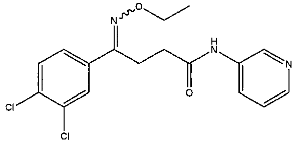 | 4-(3,4-dichlorophényle)-4-éthoxyimino-*N*-(3-pyridyle)butanamide |
| **7** | 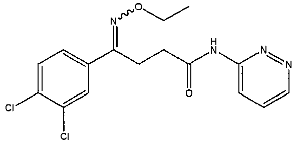 | 4-(3,4-dichlorophényle)-4-éthoxyimino-*N*-pyridazine-3-yl-butanamide |
| **8** | 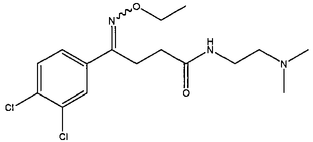 | 4-(3,4-dichlorophényle)-*N*-(2-diméthylaminoéthyle)-4-éthoxyimino-butanamide |
| **9** | 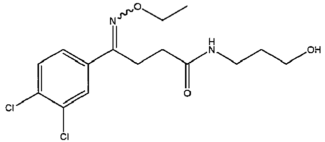 | 4-(3,4-dichlorophényle)-4-éthoxyimino-N-(3-hydroxypropyle)butanamide |
| **10** | 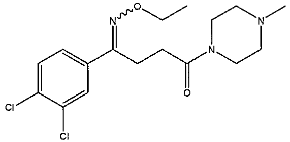 | 4-(3,4-dichlorophényle)-4-éthoxyimino-1-(4-méthylpipérazine-1-yl)butan-1-one |

En effet, la demande de brevet WO 2010011302 décrit la synthèse des composés **1** à **10** couverts par la formule générale (I) et leurs applications dans le domaine du traitement des maladies neurodégénératives sans toutefois décrire d'activité sur l'atrophie musculaire. Ainsi donc, les inventeurs ont découverts de façon surprenante que ces composés connus avaient une activité dans le traitement et/ou la prévention des pathologies liées à l'atrophie musculaire chez le mammifère.

Dans le cadre de la présente invention on entend par «groupe aryle », un cycle aromatique ayant 5 à 8 atomes de carbones ou plusieurs cycles aromatiques fusionnés ayant 5 à 14 atomes de carbones. En particulier, les groupes aryles peuvent être des groupes monocycliques ou bicycliques, de préférence phényle ou naphthyle. Avantageusement il s'agit d'un groupe phényle (Ph).

Dans le cadre de la présente invention on entend par « groupe hétéroaryle », tout groupe aromatique hydrocarboné de 3 à 9 atomes contenant un ou plusieurs hétéroatomes, en particulier un ou deux, tels que par exemple des atomes de soufre, azote ou oxygène, en particulier un ou plusieurs atomes d'azote. L'hétéroaryle selon la présente invention peut être constitué par un ou plusieurs cycles fusionnés. Des exemples de groupes hétéroaryle sont les groupes furyle, isoxazyle, pyridyle, thiazolyle, pyrimidyle, pyridazinyle, benzimidazole, benzoxazole, benzothiazole, pyrazole. Avantageusement le groupe hétéroaryle est choisi parmi les groupes pyridazinyle, pyrazole et pyridyle.

Dans le cadre de la présente invention on entend par «atome d'halogène» tout atome d'halogène, avantageusement choisi parmi Cl, Br, I ou F, en particulier choisi parmi F, Cl ou Br, en particulier F ou Cl, plus particulièrement Cl.

Dans le cadre de la présente invention on entend par « groupe alkyle en C₁-C₆ », tout groupe alkyle de 1 à 6 atomes de carbones, linéaire ou ramifié, en particulier, les groupes méthyle, éthyle, n-propyle, iso-propyle, n-butyle, iso-butyle, sec-butyle, t-butyle, n-pentyle, n-hexyle. Avantageusement il s'agit d'un groupe méthyle (Me), éthyle (Et), iso-propyle (iPr) ou t-butyle (tBu), en particulier d'un groupe méthyle, éthyle ou iso-propyle, plus particulièrement d'un groupe méthyle ou éthyle.

Dans le cadre de la présente invention on entend par « groupe cycloalkyle en C₃-C₆», tout cycle saturé et hydrocarboné comprenant de 3 à 6 atomes de carbones, en particulier, le groupe cyclopropyle, cyclobutyle, cyclopentyle ou cyclohexyle. Avantageusement il s'agit d'un groupe cyclopentyle ou cyclohexyle.

Dans le cadre de la présente invention on entend par « groupe hétérocyclique », tout groupe hydrocarboné cyclique saturé de 3 à 9 atomes contenant un ou plusieurs hétéroatomes, tels que par exemple des atomes de soufre, azote ou oxygène, en particulier des atomes d'azote et d'oxygène, plus particulièrement un ou plusieurs atomes d'azote. Le groupe hétérocyclique selon la présente invention peut être constitué par un ou plusieurs cycles fusionnés. Des exemples de groupes hétérocycliques sont les groupes tétrahydrofurane, tétrahydropyrane, pyrrolidine, pipérazine, pipéridine, thiolane, oxirane, oxane, thiane, thiazolidine, morpholine. Avantageusement le groupe hétérocyclique est choisi parmi les groupes tétrahydropyrane, pipéridine, pyrrolidine, pipérazine et morpholine.

Dans le cadre de la présente invention on entend par «pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

Dans le cadre de la présente invention on entend par « sels pharmaceutiquement acceptables d'un composé » des sels qui sont pharmaceutiquement acceptables, comme défini ici, et qui possèdent l'activité pharmacologique souhaitée du composé parent. De tels sels comprennent :
(1) les sels d'addition d'acide formés avec des acides minéraux tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires ; ou
(2) les sels formés lorsqu'un proton acide présent dans le composé parent soit est remplacé par un ion métallique, par exemple un ion de métal alcalin, un ion de métal alcalino-terreux ou un ion d'aluminium ; soit se coordonne avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Dans le cadre de la présente invention on entend par « solvate d'un composé », tout composé obtenu par addition d'une molécule de solvant inerte sur le composé selon l'invention, le solvate se formant en raison de leur force d'attraction mutuelle. Les solvates sont par exemple des alcoolates du composé. Un hydrate est un solvate dans lequel le solvant inerte utilisé est l'eau. Il peut être mono, di ou trihydraté.

Dans le cadre de la présente invention on entend par «tautomère» tout isomère de constitution des composés selon la présente invention qui sont interconvertibles par la réaction chimique réversible appelée tautomérisation. Dans la plupart des cas, la réaction se produit par migration d'un atome d'hydrogène accompagnée d'un changement de localisation d'une double liaison. Dans une solution d'un composé capable de tautomérisation, un équilibre entre les 2 tautomères se crée. Le rapport entre tautomères est alors fonction du solvant, de la température et du pH. La tautomérie est donc la transformation d'un groupement fonctionnel en un autre, le plus souvent par déplacement concomitant d'un atome d'hydrogène et d'une liaison π (liaison double ou triple). Des tautomères courants sont par exemple les paires aldéhydes / cétones - alcools ou plus précisément énol; amides - acides imidiques; lactames - lactimes; imines - énamines; énamines - énamines. En particulier il peut inclure une tautomérie cycle - chaîne qui a lieu lorsque le mouvement du proton est accompagné par la transformation d'une structure ouverte à un cycle.

Les dérivés selon l'invention peuvent être administrés sous la forme d'une composition pharmaceutique comprenant un excipient pharmaceutiquement acceptable.

Ces compositions peuvent être formulées pour l'administration aux mammifères, y compris l'homme. La posologie varie selon le traitement et selon l'affection en cause. Ces compositions pharmaceutiques sont adaptées pour une administration par n'importe quelle voie appropriée, par exemple par voie orale (incluant buccale et sublinguale), par voie rectale, nasale, topique (incluant transdermique), vaginale, intraoculaires ou parentérale (incluant sous-cutanée, intramusculaire ou intraveineuse). Avantageusement les compositions pharmaceutiques sont adaptées pour une administration par voie orale. Ces formulations peuvent être préparées en utilisant tous les procédés connus par l'homme du métier en combinant les ingrédients actifs avec les excipients pharmaceutiquement acceptables appropriés.

Les formes unitaires d'administrations appropriées par voie orale comprennent les comprimés, les gélules, les poudres, les granules et les solutions ou suspensions orales dans des liquides aqueux ou non aqueux, les mousses comestibles ou alimentaires, ou les émulsions liquides eau-dans-huile ou huile-dans-eau. Lorsque l'on prépare une composition solide sous forme de comprimé, on mélange l'ingrédient actif principal, avantageusement sous forme de poudre, avec un excipient pharmaceutique approprié tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif, avantageusement sous forme de poudre, avec un diluant et en versant le mélange obtenu dans des gélules molles ou dures, en particulier des gélules de gélatine. Des lubrifiants tels que par exemple le talc, le stéarate de magnésium, le stéarate de calcium ou le polyéthylène glycol sous forme solide peuvent être ajoutés dans la composition avant sa mise en gélule. Un désintégrant ou un solubilisant tels que par exemple le carbonate de calcium ou le carbonate de sodium peuvent également être ajoutés afin d'améliorer la disponibilité du médicament après la prise de la gélule.

En outre on peut ajouter si nécessaire dans le mélange, des liants, des lubrifiants et des désintégrant appropriés de même que des colorants. Les liants appropriés peuvent être par exemple l'amidon, la gélatine, les sucres naturels tels que par exemple le glucose ou le bêta-lactose, des agents sucrants fabriqués à partir de maïs, du caoutchouc synthétique ou naturel tel que par exemple l'acacia ou l'alginate de sodium, de la carboxyméthylcellulose, du polyéthylène glycol, des cires et similaires. Les lubrifiants utilisables dans ces formes de dosage incluent l'oléate de sodium, le stéarate de sodium, le stéarate de magnésium, le benzoate de sodium, l'acétate de sodium, le chlorure de sodium et similaires. Les désintégrant incluent l'amidon, la méthylcellulose, l'agar, la bentonite, la gomme de xanthane et similaires. Les comprimés sont formulés par exemple par préparation d'un mélange de poudre, granulation ou pressage à sec du mélange, addition d'un lubrifiant et d'un désintégrant et pressage du mélange pour donner les comprimés. Un mélange de poudre est préparé par mélange du principe actif additionné de façon appropriée avec un diluant ou une base et optionnellement avec un liant tel que par exemple la carboxyméthylcellulose, l'alginate, la gélatine ou la polyvinylpyrrolidone, un retardant de dissolution tel que par exemple la paraffine, un accélérant d'absorption tel que par exemple un sel quaternaire et/ou un absorbant tel que par exemple la bentonite, le kaolin ou le phosphate dicalcique. Les mélanges de poudres peuvent être granulés par mouillage avec un liant tel que par exemple un sirop, une pâte d'amidon, du mucilage d'acacia ou des solutions de cellulose ou de matériaux polymères et pressage à travers un tamis. Les granules peuvent être lubrifiés par addition d'acide stéarique, de sel de stéarate, de talc ou d'une huile minérale de façon à éviter qu'ils collent aux moules permettant la fabrication des comprimés. Le mélange lubrifié est alors pressé pour donner les comprimés. Une couche protectrice opaque ou transparente consistant en une couche shellac, une couche de sucre ou de matériaux polymères est éventuellement présente. Des colorants peuvent être ajoutés à ces enrobages façon à les différencier des autres comprimés.

Une préparation sous forme de sirop ou d'élixir peut contenir l'ingrédient actif conjointement avec un édulcorant, un antiseptique, ainsi qu'un agent donnant du goût et un colorant approprié. En général les préparations de sirop sont obtenues par dissolution du composé dans une solution aqueuse avec un agent donnant du goût approprié alors que les élixirs sont préparés en utilisant un véhicule alcoolique non toxique.

Les poudres ou les granules dispersibles dans l'eau peuvent contenir l'ingrédient actif en mélange avec des agents de dispersion ou des agents mouillants, ou des agents de mise en suspension, tels que par exemple des alcools isostéaryle éthoxylés et des éthers de sorbitol polyoxyéthylène, et de même qu'avec des correcteurs du goût ou des édulcorants.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Le principe actif peut être formulé également sous forme de microcapsules, éventuellement avec un ou plusieurs supports additifs.

Les compositions pharmaceutiques adaptées pour une administration par voie topique peuvent être formulées sous forme de crème, d'onguent, de suspension, de lotion, de poudre, de solution, de pâte, de gel, de spray, d'aérosols ou d'huiles.

Des compositions pharmaceutiques adaptées à l'administration par voie nasale dans lequel l'excipient support est à l'état solide comprennent des poudres ayant des tailles de particules par exemple dans la gamme de 20 à 500 microns, administrées par inhalation à partir d'un récipient contenant la poudre disposé à proximité du nez.

Les formulations pharmaceutiques adaptées à l'administration par voie vaginale peuvent assez être administrées sous forme de tampon, crème, gel, pâtes, mousse ou spray.

Dans un mode de réalisation avantageux, la composition pharmaceutique selon la présente invention comprend en outre un autre agent actif, ayant avantageusement un effet complémentaire ou synergique.

Ce second agent actif peut être administré dans la même composition pharmaceutique que le dérivé de formule (I) de la présente invention. Il peut être aussi administré séparément, soit au même moment soit de façon étalée dans le temps.

Les dérivés selon l'invention sont fabriqués par des méthodes bien connues de l'homme du métier et en particulier les procédés de préparation des composés 1 à 10 sont décrits dans l'art antérieur (WO 2010011302).

L'invention sera mieux comprise à la lumière de la description des figures et des exemples qui suivent qui sont donnés à titre indicatif.

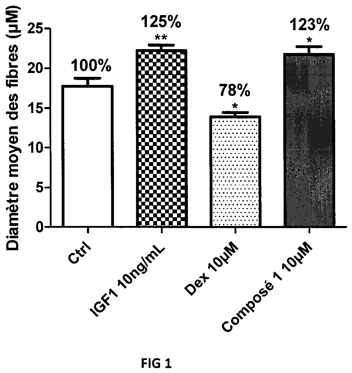

La figure 1 représente la visualisation du diamètre des fibres musculaires (en µm) en présence du véhicule (contrôle : Ctrl), du composé **1** à une concentration de 10 µM, de la dexamethasone (DEX) à une concentration de 10 µM et de l'IGF1 à une concentration de 10 ng/ml.

### CASCADE DE SCREENING

Le développement du test de criblage a été initié à partir des travaux de la littérature et basé sur les caractéristiques de la pathologie de la Sarcopénie. Au niveau physiopathologique, cette maladie se caractérise par une diminution de la synthèse protéique et une augmentation de la protéolyse. Le triage des composés cités ci-dessus a donc été effectué sur des tests permettant d'évaluer la capacité des molécules chimiques à inhiber l'expression génique de la myostatine et leur capacité à augmenter la synthèse protéique dans des cellules musculaires. De plus, nous avons montré que certains produits de la liste ci-dessus augmentaient le diamètre des myotubes de cellules C2C12.

### Protocoles :

### Mesure de l'expression de la myostatine dans des cellules C2C12 :

Les cellules myoblastiques C2C12 (ATCC CRL-1772) sont ensemencées en plaques 24 puits à la densité de 30000 cellules par puits et cultivées dans un milieu DMEM (Dulbecco's Modified Eagle Medium) contenant du glucose à raison de 4,5 g/L et supplémenté en sérum de veau foetal (10%) et en antibiotiques (pénicilline et streptomycine). Quarante huit heures plus tard, les myoblastes sont induits en différenciation par privation partielle en sérum (2% au lieu de 10%) pendant 5 jours. Les cellules sont ensuite placées dans un milieu appauvri en glucose (DMEM contenant 1 g/L de glucose) et dépourvu de sérum en présence des molécules à tester ou des références (IGF-1 à la concentration de 100 ng/mL) pendant 6 h. A la fin de l'expérience, les ARN messagers (ARNm) sont extraits en utilisant la méthodologie classique à base de phénol et chloroforme. Brièvement, les cellules sont lysées dans une solution de trizol (Sigma T9424) contenant un acide fort et du phénol. Les ARNm sont séparés des protéines par ajout de chloroforme puis centrifugation. Ils sont ensuite précipités dans l'isopropanol pour ensuite être suspendus à la concentration de 1µg/µL dans une eau ultra pure débarrassée des RNases et DNAses. 1µg d'ARNm est alors converti par rétro transcription en ADN complémentaire par l'enzyme AMV en présence d'une amorce et d'un mélange de nucléotides selon le protocole donné par le fournisseur (Applied Biosystems 4368814). L'expression génique est étudiée par réaction en chaine initiée par une enzyme polymérase et couramment nommé PCR dans des conditions quantitative, d'où le nom précis de qPCR. Les qPCR sont réalisées sur un 7900HT Fast real-Time PCR detection system (Applied Biosystems). Les conditions de programmation sont standard et consistent en 1 cycle à 95°C pendant 15 min, suivi de 40 cycles à 95°C pendant 15 s et à 60 °C pendant 1 min et on termine par une étape de courbe de fusion entre 60°C et 95°C. Les échantillons analysés contiennent tous 100 ng d'ADNc, un tampon de qPCR incluant l'enzyme, le mélange d'oligonucléotides et l'intercalant (le sybergreen), et le couple d'amorces spécifiques du gène étudié, stratégiquement choisi entre deux séquences exoniques et à la concentration finale de 200 nM. Des sondes fluorescentes se fixent sur l'ADN double brin et ne fluorescent qu'une fois fixées à l'ADN. Un seuil de fluorescence est établi par le programme de la machine. Lorsque la quantité d'ADN permet à la sonde fluorescente de dépasser ce seuil, on obtient un numéro de cycle PCR appelé "Ct" pour "Cycle Threshold" ou cycle seuil. C'est cette valeur qui est à la base des calculs pour quantifier l'ADN de façon relative. On établit un rapport R entre la quantité d'ADN de départ d'un échantillon et celle d'un témoin, qui n'a pas subi de traitement (soit R=2^{-(Ct échantillon - Ct témoin)}) et cette mesure sera rapportée à celle d'un gène ménage connu pour ne pas être modulé par le traitement (soit 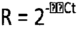 ).

Les amorces utilisées sont consignées dans le tableau suivant :
Amorces utilisées pour évaluer les modifications d'expression génique

| Gène | Séquence 5' → 3' | Nb de bases | Tm | N° d'accession |
|---|---|---|---|---|
| Myostatine d | GAGTCTGACTTTCTAATGCAAG | 21 | 62 | souris: NM_010834 |
| Myostatine ind | TGTTGTAGGAGTCTTGACGG | 20 | 60 | rat: AF019624 |
| Atrogine d | AGAGTCGGCAAGTCTGTGCT | 20 | 62 | souris: AF441120 |
| Atrogine ind | GTGAGGCCTTTGAAGGCAG | 19 | 60 | humain: NM_058229 |
| beta-actine d | CTCTAGACTTCGAGCAGGAG | 20 | 62 | souris: X03672 |
| beta-actine ind | GGTACCACCAGACAGCACT | 19 | 60 | |

### Synthèse protéique

Les cellules sont comptées et ensemencées à la densité de 20000 cellules par puits en plaque 24 puits dans un milieu DMEM contenant du glucose à raison de 4,5 g/L et supplémenté en sérum de veau foetal (10 %) et en antibiotiques (pénicilline et streptomycine). Quarante huit heures plus tard, les myoblastes sont induits en différenciation par privation partielle en sérum (2% au lieu de 10 %) pendant 5 jours. Les cellules sont ensuite placées dans un milieu sans glucose ni leucine (milieu Krebs) pendant 1h à 37°C puis incubées 2h30 en présence des produits à tester ou référence (IGF-1, 100 ng/mL) dans un milieu DMEM sans sérum contenant de la leucine radio marquée 2,5 µCi/mL. A la fin de l'incubation, les surnageants sont éliminés et les cellules sont lysées dans une solution de NaOH 0,1N pendant 30 min. La radioactivité est mesurée dans la fraction cellulaire et la quantité de protéines totale est déterminée par dosage selon la méthode Lowry. Chaque condition est évaluée au minimum en n = 6; l'IGF-1, 100 ng/mL est notre témoin pour stimuler la synthèse protéique. Les résultats sont exprimés en cpm/µg de protéines après 2h30 d'incubation ou en pourcentage par rapport à la condition contrôle.

### Evaluation du diamètre des fibres musculaires

Les cellules myoblastiques C2C12 (ATCC CRL-1772) sont ensemencées en plaques 8 puits traitées au glycérol, à la densité de 10 000 cellules par puits et cultivées dans un milieu DMEM contenant du glucose à raison de 4,5 g/L et supplémenté en sérum de veau foetal (10%) et en antibiotiques (pénicilline et streptomycine).

Quarante huit heures plus tard, les myoblastes sont induits en différenciation par privation partielle en sérum (2 % au lieu de 10 %) pendant 3 jours. Les cellules sont ensuite placées dans un milieu appauvri en glucose (DMEM contenant 1 g/L de glucose) et dépourvu de sérum en présence des molécules à tester ou des références (IGF-1 à la concentration de 10 ng/mL ou dexamethasone 10 µM) pendant 3 jours. A la fin de la culture, les cellules sont rincées et fixées à l'aide d'une solution Glutaraldehyde 2,5% / Triton 0,1% pendant 1h à température ambiante. Le tapis cellulaire est recouvert de DAPI (marquage fluorescent du noyau cellulaire). Après stockage à l'obscurité pendant 16h au froid, les lames sont observées sous microscope à fluorescence (Carl Zeiss, AxioVert 200) et les images analysées à l'aide du logiciel Axiovision 4.1 pour mesurer le diamètre des fibres.

### Résultats :

### • Effets sur l'expression de la myostatine (tableau 1)

Pour les effets sur l'expression de la myostatine, les résultats sont exprimés en pourcentage d'expression génique de la myostatine dans les cellules en contact avec les molécules rapporté à l'expression dans les cellules contrôle. Le produit est supposé actif si ce ratio est inférieur ou proche de 70%, valeur moyenne constaté de l'IGF-1 à la concentration de 100 ng/mL dans ce test, considéré comme référence.

**Tableau 1**

| **Numéro du composé** | **Concentration du composé** |
|---|---|
| | **0,5 µM** |
| **1** | 71% |
| **3** | 41% |

Les composés 1 et 3 inhibent de façon significative l'expression de la myostatine.

### • Effets sur la synthèse protéique via la phosphorylation de S6K1 (tableau 2)

Pour les effets sur la synthèse protéique, les résultats sont exprimés en pourcentage d'augmentation de la phosphorylation de S6K dans les cellules musculaires. Ce pourcentage est estimé significatif lorsqu'il est supérieur à 120%, valeur moyenne constaté de l'IGF-1 à la concentration de 100 ng/mL dans ce test, considéré comme référence.

**Tableau 2**

| **Numéro du composé** | **synthèse protéique** | | |
|---|---|---|---|
| | **0,5 µM** | **5 µM** | **50 µM** |
| **1** | 134% | 142% | 151% |

Le composé **1** augmente de façon significative la synthèse protéique dans les cellules de muscle dès la concentration de 0,5 µM.

### • Effet du composé 1 sur le diamètre des fibres musculaires (figure 1)

Les cellules C2C12 sont différenciées et traitées avec le composé 1 les trois derniers jours de la différentiation. Les résultats sont représentés sur la figure 1.

Comme attendu, le traitement à l'IGF-1 accroit de manière significative le diamètre des myotubes et par contraste la dexamethasone décroit significativement ce paramètre. Le diamètre des myotubes est significativement augmenté après traitement avec le composé **1** (figure 1).

### BIBLIOGRAPHIE

Bass J et al., Domest Anim Endocrinol, 1999, 17(2-3) : 1991-197
Baumgartner R N et al., The journals of gerontology. Series A, Biological sciences and medical sciences 1998, 53(4): M264-74
Chai RJ et al., PLoS One 2011, 6: e28090
Farnfield MM et al., Appl Physiol Nutr Metab 2012, 37: 21-30
Frontera W R et al., Journal of applied physiology (Bethesda, Md. : 1985) 1991, 71(2): 644-50
Hasselgren PO et al., Biochemical and Biophysical Research Communications 2002, 290(1): 1-10
Lee SJ. Immunol Endocr Metab Agents Med Chem. 2010, 10: 183-194
Léger B et al., Rejuvenation Res 2008, 11(1): 163-175
Li ZB et al., J. Biol. Chem. 2008, 283(28): 19371-19378
Lloyd BD et al., J Gerontol A Biol Sci Med Sci. 2009 May, 64(5): 599-609
McPherron AC et al., Proc. Natl. Acad. Sci. USA 1997, 94: 12457-12461
McPherron AC et al., J Clin Invest. 2002, 109: 595-601
Morley J E et al., Current Pharmaceutical Design 2007, 13(35): 3637-3647
O'Neill ED et al., Age (Dordr) 2010, 32: 209-22
Purintrapiban J et al., Comparative Biochemistry and Physiology B-Biochemistry & Molecular Biology 2003, 136(3): 393-401
Thies RS et al., Growth Fact. 2001, 18: 251-259
Wolfman NM et al., Proc. Natl. Acad. Sci. USA 2003, 100: 15842-15846
WO 2010011302

## Revendications

1. Dérivé de formule générale I suivante : 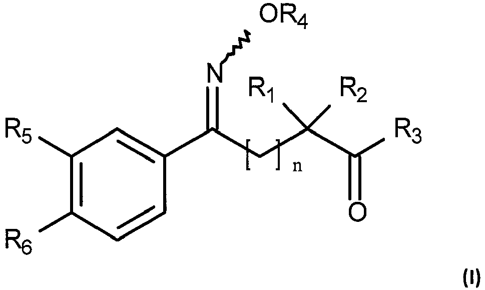 dans laquelle
- **R¹** et **R²** représentent indépendamment l'un de l'autre un atome d'hydrogène ou un groupe alkyle en C₁-C₆, avantageusement méthyle ;
- **n** est un nombre entier compris entre 1 et 2, avantageusement **n**=1 ;
- **R³** représente
- un groupe -OH;
- un groupe-O(alkyle en C₁-C₆), avantageusement-OEt ou -OiPr;
- un groupe-O(cycloalkyle en C₃-C₆), avantageusement-O-cyclopentyle;
- un groupe -O(hétérocyclique en C₃-C₇), avantageusement-O-tétrahydropyran-4 yl;
- un groupe -O-hétéroaryle, avantageusement -O-pyridyle ;
- un groupe -O-aryle, avantageusement-O-phényle ;
- un groupe - O(alkyle en C₁-C₆)aryle, avantageusement -O-benzyle ;
- un groupe -N**R⁷R⁸**, dans lequel
**R⁷** représente un atome d'hydrogène ou un groupe alkyle en C₁-C₆ **et R⁸** représente
- un atome d'hydrogène ;
- un groupe hétéroaryle, avantageusement contenant un ou plusieurs atomes d'azote;
- un groupe alkyle en C₁-C₆, avantageusement un groupe éthyle, éventuellement substitué par un groupe -N**R⁹R¹⁰** dans lequel **R⁹** et **R¹⁰** représentent indépendamment l'un de l'autre un atome d'hydrogène ou un groupe alkyle en C₁-C₆, avantageusement un groupe méthyle ;
- un groupe alkyle en C₁-C₆, avantageusement un groupe éthyle, éventuellement substitué par un groupe -O**R¹¹** dans lequel **R¹¹** représente un atome d'hydrogène ou un groupe alkyle en C₁-C₆, avantageusement un groupe méthyle ;
**ou R⁷** et **R⁸** forment avec l'atome d'azote qui les portent un hétérocycle, en particulier une pyrrolidine, une pipéridine, une morpholine, une pipérazine, l'hétérocycle étant éventuellement substituée par un groupe alkyle en C₁-C₆, avantageusement un groupe méthyle ;
- **R⁴** représente un atome d'hydrogène ou un groupe alkyle en C₁-C₆, en particulier -Me ou -Et ;
- **R⁵** et **R⁶** représentent indépendamment l'un de l'autre un atome d'hydrogène, un atome d'halogène, avantageusement Cl, ou un groupe choisi parmi :
un groupe -CN ;
un groupe -OH ;
un groupe -O(alkyle en C₁-C₆), avantageusement -OMe, le groupe alkyle étant éventuellement substitué par un ou plusieurs atomes d'halogène, avantageusement F;
un groupe alkyle en C₁-C₆, avantageusement méthyle, éventuellement substitué par un ou plusieurs atomes d'halogène, avantageusement F ;
et un groupe -O(cycloalkyle en C₃-C₆), avantageusement O-cyclopropyle ; ;
ou un énantiomère, diastéréoisomère, hydrate, solvate, tautomère, mélange racémique ou sel pharmaceutiquement acceptable de celui-ci
pour utilisation à titre de médicament destiné au traitement et/ou à la prévention de l'atrophie musculaire chez les mammifères et/ou pour limiter l'atrophie musculaire chez les mammifères et/ou pour favoriser l'accroissement musculaire des mammifères faisant de l'exercice et visant à augmenter la masse et la qualité musculaire, en prévention de l'apparition de symptômes sarcopéniques liés à l'âge ou en rééducation suite à une perte musculaire,
l'atrophie musculaire étant liée à l'âge et/ou aux conséquences d'un traitement médicamenteux et/ou à une immobilisation et/ou à la cachexie.

2. Dérivé pour utilisation selon la revendication **1, caractérisé en ce que R⁵** et **R⁶** représentent indépendamment l'un de l'autre un atome d'halogène, avantageusement **R⁵** et **R⁶** sont des atomes de chlores.

3. Dérivé pour utilisation selon l'une quelconque des revendications 1 à 2, **caractérisé en ce que R⁴** représente un groupe alkyle en C₁-C₆, avantageusement -éthyle.

4. Dérivé pour utilisation selon l'une quelconque des revendications 1 à 3, **caractérisé en ce que n** = 1 ;

5. Dérivés pour utilisation selon l'une quelconque des revendications 1 à 4, **caractérisé en ce que R³** représente un groupe -OH ou un groupe -O(alkyle en C₁-C₆), avantageusement -OEt ou -OiPr.

6. Dérivé pour utilisation selon l'une quelconque des revendications 1 à 5, **caractérisé en ce que R¹** et **R²** représentent tous les deux un atome d'hydrogène.

7. Dérivé pour utilisation selon l'une quelconque des revendications 1 à 6, **caractérisé en ce qu'**il est choisi parmi les composés suivants.
(1) acide 4-(3,4-dichlorophényle)-4-éthoxyimino-butanoique;
(2) acide 4-(3,4-dichlorophényle)-4-méthoxyimino-butanoique;
(3) 4-(3,4-dichlorophényle)-4-éthoxyimino-butanoate d'isopropyle;
(4) acide 5-(3,4-dichlorophényle)-5-éthoxyimino-pentanoique;
(5) 4-(3,4-dichlorophényle)-4-éthoxyimino-*N*-(2-pyridyle)butanamide;
(6) 4-(3,4-dichlorophényle)-4-éthoxyimino-*N*-(3-pyridyle)butanamide;
(7) 4-(3,4-dichlorophényle)-4-éthoxyimino-*N*-pyridazine-3-yl-butanamide;
(8) 4-(3,4-dichlorophényle)-*N*-(2-diméthylaminoéthyle)-4-éthoxyimino-butanamide;
(9) (4E)-4-(3,4-dichlorophényle)-4-éthoxyimino-N-(3-hydroxypropyle)butanamide ;
(10) 4-(3,4-dichlorophényle)-4-éthoxyimino-1-(4-méthylpiperazine-1-yl)butan-1-one;

8. Dérivé pour utilisation selon l'une quelconque des revendications 1 à 7, **caractérisé en ce que** l'atrophie musculaire liée à l'âge est la pré-sarcopénie, la sarcopénie ou la sarcopénie sévère.

9. Dérivé pour utilisation selon l'une quelconque des revendications 1 à 8, **caractérisé en ce que** le mammifère est l'homme.

## Patentansprüche

1. Derivat mit der folgenden allgemeinen Formel I: 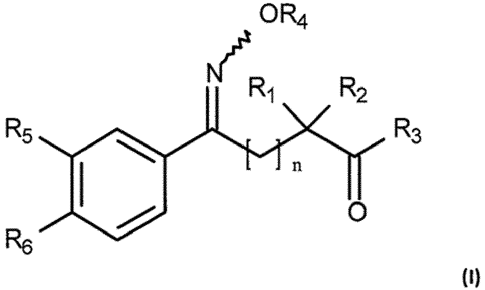 wobei:
- R¹ und R² unabhängig voneinander ein Wasserstoffatom oder eine C₁-C₆-Alkylgruppe, vorteilhaft Methyl, darstellen;
- n eine ganze Zahl zwischen 1 und 2 ist, wobei vorteilhaft n = 1;
- R³ darstellt:
- eine Gruppe -OH;
- eine -O(C₁-C₆-Alkylgruppe), vorteilhaft -OEt oder -OiPr;
- eine -O(C₃-C₆-Cycloalkylgruppe), vorteilhaft -O-Cyclopentyl;
- eine -O(C₃-C₇-heterocyclische) Gruppe, vorteilhaft -O-Tetrahydropyran-4-yl;
- eine -O-Heteroarylgruppe, vorteilhaft -O-Pyridyl;
- eine O-Arylgruppe, vorteilhaft -O-Phenyl;
- eine -O(C₁-C₆-Alkyl)-arylgruppe, vorteilhaft -O-Benzyl;
- eine Gruppe -NR⁷R⁸, wobei:
R⁷ ein Wasserstoffatom oder eine C₁-C₆-Alkylgruppe darstellt, und R⁸ darstellt:
- ein Wasserstoffatom;
- eine Heteroarylgruppe, vorteilhaft enthaltend ein oder mehrere Stickstoffatome;
- eine C₁-C₆-Alkylgruppe, vorteilhaft eine Ethylgruppe, gegebenenfalls substituiert durch eine Gruppe -NR⁹R¹⁰, wobei R⁹ und R¹⁰ unabhängig voneinander ein Wasserstoffatom oder eine C₁-C₆-Alkylgruppe, vorteilhaft eine Methylgruppe, darstellen;
- eine C₁-C₆-Alkylgruppe, vorteilhaft eine Ethylgruppe, gegebenenfalls substituiert durch eine Gruppe -OR¹¹, wobei R¹¹ ein Wasserstoffatom oder eine C₁-C₆-Alkylgruppe, vorteilhaft eine Methylgruppe, darstellt;
oder R⁷ und R⁸ mit dem Stickstoffatom, das diese tragen, einen Heterocyclus bilden, insbesondere ein Pyrrolidin, ein Piperiden, ein Morpholin, ein Piperazin, wobei der Heterocyclus gegebenenfalls substituiert ist durch eine C₁-C₆-Alkylgruppe, vorteilhaft eine Methylgruppe;
- R⁴ ein Wasserstoffatom oder eine C₁-C₆-Alkylgruppe, insbesondere -Me oder -Et, darstellt;
- R⁵ und R⁶ unabhängig voneinander ein Wasserstoffatom, ein Halogenatom, vorteilhaft Cl, oder eine Gruppe darstellen, ausgewählt aus:
einer Gruppe -CN;
einer Gruppe -OH;
einer -O(C₁-C₆-Alkylgruppe), vorteilhaft -OMe, wobei die Alkylgruppe gegebenenfalls substituiert ist durch ein oder mehrere Halogenatome, vorteilhaft F;
einer C₁-C₆-Alkylgruppe, vorteilhaft Methyl, gegebenenfalls substituiert durch ein oder mehrere Halogenatome, vorteilhaft F;
und einer -O(C₃-C₆-Cycloalkylgruppe), vorteilhaft O-Cyclopropyl;
oder ein Enantiomer, Diastereoisomer, Hydrat, Solvat, Tautomer, eine racemische Mischung oder ein pharmazeutisch annehmbares Salz desselben,
zur Verwendung als Medikament, das zur Behandlung und/oder zur Prävention einer Muskelatrophie bei Säugern, und/oder zur Eindämmung einer Muskelatrophie bei Säugern, und/oder zur Förderung des Muskelwachstums bei trainierenden Säugern bestimmt ist, und die Muskelmasse und -qualität erhöhen soll, zur Prävention des Auftretens sarkopenischer Symptome, die mit dem Alter oder mit einer Rehabilitation nach einem Muskelschwund verbunden sind, wobei die Muskelatrophie mit dem Alter und/oder mit den Folgen einer medikamentösen Behandlung und/oder mit einer Immobilisation und/oder mit einer Kachexie verbunden ist.

2. Derivat zur Verwendung nach Anspruch 1, **dadurch gekennzeichnet, dass** R⁵ und R⁶ unabhängig voneinander ein Halogenatom darstellen, wobei R⁵ und R⁶ vorteilhaft Chloratome sind.

3. Derivat zur Verwendung nach einem der Ansprüche 1 bis 2, **dadurch gekennzeichnet, dass** R⁴ eine C₁-C₆-Alkylgruppe, vorteilhaft -Ethyl, darstellt.

4. Derivat zur Verwendung nach einem der Ansprüche 1 bis 3, **dadurch gekennzeichnet, dass** n = 1.

5. Derivat zur Verwendung nach einem der Ansprüche 1 bis 4, **dadurch gekennzeichnet, dass** R³ eine Gruppe -OH oder eine -O(C₁-C₆-Alkylgruppe), vorteilhaft -OEt oder -OiPr, darstellt.

6. Derivat zur Verwendung nach einem der Ansprüche 1 bis 5, **dadurch gekennzeichnet, dass** R¹ und R² beide ein Wasserstoffatom darstellen.

7. Derivat zur Verwendung nach einem der Ansprüche 1 bis 6, **dadurch gekennzeichnet, dass** dieses aus den folgenden Verbindungen ausgewählt ist:
(1) 4-(3,4-Dichlorphenyl)-4-ethoxyiminobutansäure;
(2) 4-(3,4-Dichlorphenyl)-4-methoxyiminobutansäure;
(3) Isopropyl-4-(3,4-dichlorphenyl)-4-ethoxyiminobutanoat;
(4) 5-(3,4-Dichlorphenyl)-5-ethoxyimino-pentansäure;
(5) 4-(3,4-Dichlorphenyl)-4-ethoxyimino-N-(2-pyridyl)-butanamid;
(6) 4-(3,4-Dichlorphenyl)-4-ethoxyimino-N-(3-pyridyl)-butanamid;
(7) 4-(3,4-Dichlorphenyl)-4-ethoxyimino-N-pyridazin-3-yl-butanamid;
(8) 4-(3,4-Dichlorphenyl)-N-(2-dimethylaminoethyl)-4-ethoxyiminobutanamide;
(9) (4E)-4-(3,4-Dichlorphenyl)-4-ethoxyimino-N-(3-hydroxypropyl)-butanamid;
(10) 4-(3,4-Dichlorphenyl)-4-ethoxyimino-1-(4-methylpiperazin-1-yl)-butan-1-on.

8. Derivat zur Verwendung nach einem der Ansprüche 1 bis 7, **dadurch gekennzeichnet, dass** die mit dem Alter verbundene Muskelatrophie Präsarkopenie, Sarkopenie oder schwere Sarkopenie ist.

9. Derivat zur Verwendung nach einem der Ansprüche 1 bis 8, **dadurch gekennzeichnet, dass** der Säuger ein Mensch ist.

## Claims

1. Derivative of general formula I below: 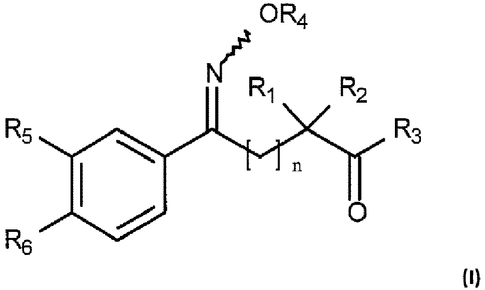 in which
- R¹ and R² independently from each another are hydrogen or (C₁-C₆) alkyl, preferably a methyl;
- n is an integer between 1 and 2, preferably n = 1;
- R³ represents
- an -OH group;
- an -O(C₁-C₆ alkyl) group, advantageously -OEt or -OiPr;
- an -O (C₃-C₆ cycloalkyl) group, advantageously an -O-cyclopentyl;
- an -O (C₃-C₇ heterocyclic) group, advantageously an -O-tetrahydropyran-4-yl;
- an -O-heteroaryl group, advantageously an -O-pyridyl;
- an -O-aryl group, advantageously an -O-phenyl;
- an -O(C₁-C₆ alkyl) aryl group, advantageously -O-benzyl;
- an -NR⁷R⁸ group, in which
R⁷ represents a hydrogen atom or a C₁-C₆ alkyl group and R⁸ represents
- a hydrogen atom;
- a heteroaryl group, advantageously containing one or more nitrogen atoms;
- a C₁-C₆ alkyl group, advantageously an ethyl group, optionally substituted with an -NR⁹R¹⁰ group in which R⁹ and R¹⁰ represent, independently of one another, a hydrogen atom or a C₁-C₆ alkyl group, advantageously a group methyl;
- a C₁-C₆ alkyl group, advantageously an ethyl group, optionally substituted with a group -OR¹¹ in which R¹¹ represents a hydrogen atom or a C₁-C₆ alkyl group, advantageously a methyl group;
or R⁷ and R⁸ form, with the nitrogen atom to which they are attached, a heterocycle, in particular a pyrrolidine, a piperidine, a morpholine, a piperazine, the heterocycle being optionally substituted with a C₁-C₆ alkyl group, advantageously a methyl group;
- R⁴ represents a hydrogen atom or a C₁-C₆ alkyl group, in particular -Me or -Et;
- R⁵ and R⁶ represent, independently from each other, a hydrogen atom, a halogen atom, advantageously Cl, or a group chosen from:
a -CN group;
an -OH group;
an -O(C₁-C₆) alkyl group, advantageously -OMe, the alkyl group being optionally substituted by one or more halogen atoms, advantageously F;
a C₁-C₆ alkyl group, advantageously methyl, optionally substituted with one or more halogen atoms, advantageously F;
an -O (C₃-C₆ cycloalkyl) group, advantageously an -O-cyclopropyl;
or an enantiomer, diastereoisomer, hydrate, solvate, tautomer, racemic mixture or pharmaceutically acceptable salt thereof
for use as a medicament for the treatment and/or prevention of muscle atrophy in mammals and/or for limiting muscular atrophy in mammals and/or to promote muscle growth in exercising mammals aimed at increasing muscle mass and quality, preventing the occurrence of symptoms of sarcopenia related to age or rehabilitation after a muscle loss, age-related muscle atrophy and/or the consequences of drug therapy and/or immobilization and/or cachexia.

2. Derivative for use according to Claim 1, **characterized in that** R⁵ and R⁶ represent independently from each other a halogen atom, preferably R⁵ and R⁶ are chlorine atoms.

3. Derivative for use according to any one of Claims 1 to 2, **characterized in that** R⁴ represents a C₁-C₆ alkyl group, advantageously -ethyl.

4. Derivative for use according to any one of Claims 1 to 3, **characterized in that** n = 1;

5. Derivatives for use according to any one of Claims 1 to 4, **characterized in that** R³ represents an -OH group or an -O(C₁-C₆ alkyl) group, advantageously -O Et or -OiPr.

6. Derivative for use according to any one of Claims 1 to 5, **characterized in that** R¹ and R² both represent a hydrogen atom.

7. Derivative for use according to any one of Claims 1 to 6, **characterized in that** it is selected from the following compounds.
(1) 4-(3,4-dichlorophenyl)-4-ethoxyimino-butanoic acid;
(2) 4-(3,4-dichlorophenyl)-4-methoxyimino-butanoic acid;
(3) Isopropyl 4-(3,4-dichlorophenyl)-4-ethoxyimino-butanoate;
(4) 5-(3,4-dichlorophenyl)-5-ethoxyimino-pentanoic acid;
(5) 4-(3,4-dichlorophenyl)-4-ethoxyimino-N-(2-pyridyl) butanamide;
(6) 4-(3,4-dichlorophenyl)-4-ethoxyimino-N-(3-pyridyl) butanamide;
(7) 4-(3,4-dichlorophenyl)-4-ethoxyimino-N-pyridazin-3-yl-butanamide;
(8) 4-(3,4-dichlorophenyl)-N-(2-dimethylaminoethyl)-4-ethoxyimino-butanamide;
(9) (4E)-4-(3,4-dichlorophenyl)-4-ethoxyimino-N-(3-hydroxypropyl) butanamide;
(10) 4-(3,4-dichlorophenyl)-4-ethoxyimino-1-(4-methylpiperazin-1-yl) butan-1-one;

8. Derivative for use according to any one of Claims 1 to 7, **characterized in that** the age-related muscular atrophy is pre-sarcopenia, sarcopenia or severe sarcopenia.

9. Derivative for use according to any one of Claims 1 to 8, **characterized in that** the mammal is man.
